Europäisches Patentamt

European Patent Office

Office européen des brevets

(11) Publication number: **0 162 697**
**A2**

(12) **EUROPEAN PATENT APPLICATION**

(21) Application number: **85303562.4**

(22) Date of filing: **21.05.85**

(51) Int. Cl.⁴: **A 61 M 1/00**

(30) Priority: **22.05.84 US 612775**

(43) Date of publication of application:
**27.11.85 Bulletin 85/48**

(84) Designated Contracting States:
**BE DE FR GB NL SE**

(71) Applicant: **SHERWOOD MEDICAL COMPANY**
**1831 Olive Street**
**St. Louis, MO 63103(US)**

(72) Inventor: **di Palma, Giorgio Giuseppe Giacomo**
**2353 East Sublette**
**Sandy Utah 84092(US)**

(72) Inventor: **George, Robert Donald**
**204 Rue Grand**
**Lake St. Louis Missouri 63367(US)**

(74) Representative: **Chettle, Adrian John et al,**
**c/o John Wyeth & Brother Limited Huntercombe Lane**
**South**
**Taplow Maidenhead Berkshire, SL6 0PH(GB)**

㊽ **Wound drainage device.**

㊼ Wound drainage apparatus is provided which includes a vacuum bottle (16) having a pierceable seal (22) and a cap (18) defining a socket (34). A connector (20) having a fluid inlet (56) is provided with a hollow spike (62) surrounded by a shield (54). The spike (62) can be inserted into the socket (34) in sliding sealing engagement therewith for piercing the seal (22) and connecting the interior of the bottle (16) with the fluid inlet (56). A pressure indicating member (69) may be provided on the connector (20) to indicate the pressure condition of the bottle (16) when the seal (22) is pierced by the spike (62).

FIG.4

WOUND DRAINAGE APPARATUS

This invention relates to wound drainage apparatus and more particularly to the type in which a sealed air-evacuated collection container is employed to collect fluid from a wound.

Wound drainage devices are often used to aid the healing process, for example, after surgical procedures. Disposable fluid collection containers or bottles that are air-evacuated and sealed are known and usually undergo a period of storage before use. Such collection containers can be connected to a wound drainage catheter without the necessity of employing a general suction source such as a hospital suction system. When one container has been filled, a fresh evacuated container is connected to the fluid drainage system, and the filled container discarded.

Such wound drainage devices have had certain problems or disadvantages associated with their use. For example, removing a filled container and connecting a new evacuated container to the drainage system has been rather complicated. In one prior art device one end of an elastic sleeve is connected to a collection container inlet or spigot and a clamp employed to maintain the sleeve closed and the negative pressure within the container. The proximal end of a tube, which is generally used to connect a catheter with the collection container, is then inserted into the open end of the elastic sleeve and the clamp removed to thereby connect the tube and catheter in fluid communication with the interior of the container. This construction requires the manipulation of tubing and clamps and in general is not a simple and reliable manner of connecting a collection container with a catheter.

Another problem of prior art drainage devices is that it is difficult to ensure that drainage fluid does not contaminate the person handling the apparatus, for example, when changing a full collection container for an empty container.

While evacuated collection containers or bottles generally maintain their negative pressure during storage it may be desirable to have an indicator showing whether a fresh container does in fact have a reduced pressure. For this reason, negative pressure indicating devices have been employed on prior art containers to indicate the pressure condition within the container. Such pressure indicating devices add to the overall cost of each container, and are disposed of with the discarded container.

Other wound drainage devices employ springs or elastic diaphragms that are actuated just prior to use to provide a suction force or negative pressure. These devices are however relatively expensive and complicated.

It is therefore an object of the present invention to provide an improved wound drainage apparatus using a sealed, air-evacuated collection container which is economical and overcomes one or more of the above mentioned problems or disadvantages.

According to the present invention there is provided an evacuated collection container having a pierceable seal over an opening therein, a socket defined about the seal, and a connector including an inlet for drainage fluid, a spike for insertion into the socket and adapted to pierce the seal to connect the interior of the container with the inlet, and a shield surrounding the spike.

The inlet of the connector may be attached to a drainage catheter and the shield grasped to insert the spike into the socket. The use of a separate sealed collection container allows the connector to be readily changed from a full container to an empty container with the minimum of effort and delay, no clamps are required and no manipulation of tubing is necessary. The shield provides a handgrip for the connector and isolates the user from drainage fluid in the vicinity of the spike.

Preferably the socket is adapted to receive the spike in sealing engagement therewith. The socket may be provided with sealing means on the internal wall thereof for co-operation with the spike; in a preferred embodiment the socket accepts the spike in sliding sealing engagement.

The shield may encircle the spike and have an axial length as great as that of the spike. Preferably the shield has an axial length greater than that of the spike. Such a construction provides optimum protection against contamination whilst still allowing free entry of the spike into the socket.

In a preferred embodiment the shield comprises a transverse wall having a sleeve depending therefrom which is adapted to surround said socket.

The connector may be a plastics moulding having an integral upstanding tubular projection defining the inlet and an integral depending projection defining the spike, the projection and spike being in fluid communication.

The spike is preferably tubular and pointed at the outer end thereof. The shield provides additional protection against injury from the pointed spike which is provided to improve effectiveness in piercing the seal of the container.

In a preferred embodiment the container is a bottle having a neck, the seal being over the opening of the neck and a resilient sleeve being placed over the neck in elastic engagement therewith and upstanding therefrom to define the socket. The dimensions of the sleeve portion defining the socket are preferably chosen to receive the spike in sliding sealing engagement.

In one embodiment the container is of plastic and the seal comprises a laminate having a metal foil layer and an underlying thermoplastic layer bonded thereto and bonded to the opening of the container.

A stopper for the socket may be integrally connected thereto, for example by a strap, and is provided to close a used container for disposal. The stopper may be moulded as part of the socket.

The apparatus may be further provided with pressure indicating means comprising a resilient bulb in fluid communication with the connector inlet. On insertion of the connector spike into a fresh evacuated collection container the bulb will collapse as an indication of the negative pressure therein so giving a visual indication of condition of the container. Such an indicator is very economical to manufacture, is simple, effective and reusable for each replacement collection container.

The connector is preferably an integral plastics moulding as hereinbefore mentioned and having an additional upstanding tubular projection over which the bulb may elastically engage.

Other aspects of the invention will be apparent from the following description of a preferred embodiment described by way of example only with reference to the accompanying drawings in which:-

Figure 1 is an elevation of wound drainage apparatus in accordance with a preferred embodiment of the present invention;

Figure 2 is an axial cross-section on an enlarged scale of the upper part of the container shown in Figure 1 in the storage condition;

Figure 3 is a plan view of the cap of Figure 2;

Figure 4 is an axial cross-section, on an enlarged scale of the upper part of the container shown in Figure 1; and

Figure 5 is a view of the underside of the connector shown in Figure 4.

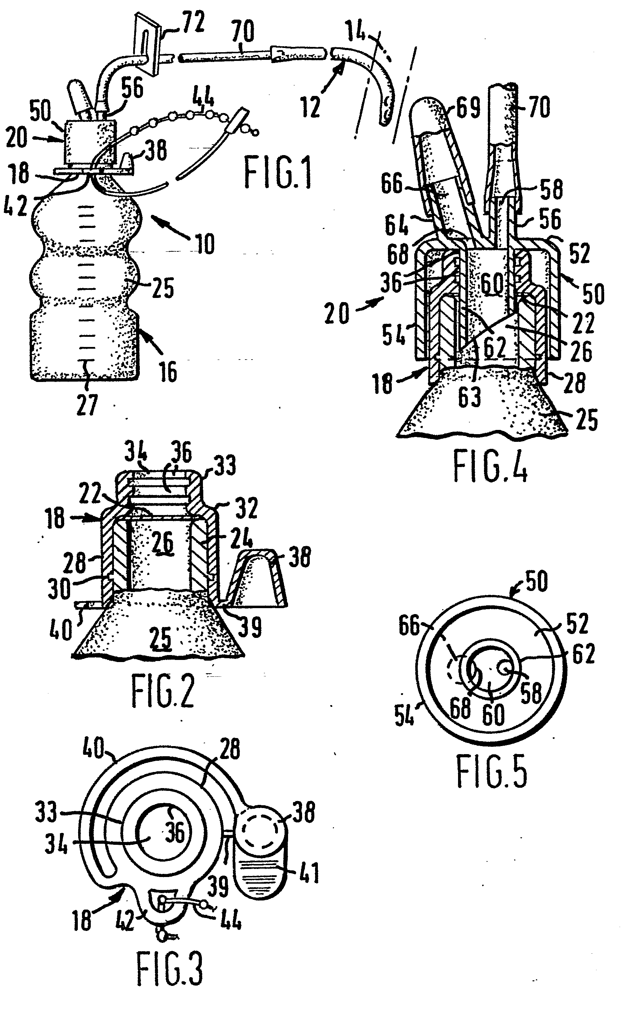

Referring now to the drawings, and more particularly to Figure 1, there is shown a wound drainage collection apparatus 10 connected to a suction catheter 12 illustrated as being disposed in a surgical wound area 14 of a patient. The distal end of the catheter has a plurality of openings for the flow of drainage gas and liquid into the catheter 12.

The apparatus 10 includes an air-evacuated collection container or vacuum bottle 16 having a cap 18 and a connector assembly 20 connecting the container 16 with the catheter 12. The partial vacuum in the bottle 16, provides a pressure differential that facilitates the flow of wound drainage fluid from the patient's wound 14 through the catheter 12 to the interior of the collection container 16 and so facilitates the healing of the wound.

The container 16 in its stored condition, as illustrated in Figure 2, has a seal 22 across the outer end of a neck 24 to maintain the negative pressure within the container. The neck 24 is integrally connected to the main drainage collection portion 25 of the container and has an axially extending bore 26. Container 16, as shown in Figure 1, has corrugations or undulations in the side walls to provide additional rigidity and strength to the container to obviate the chance of collapse due to the negative pressure within the container. The container can be made out of a relatively rigid plastic such as a thermoplastic material, for example, relatively rigid polyvinyl chloride, or polyethylene terephthalate glycol. The container may be formed of any suitable material that will maintain its shape and will not collapse either as a result of the internal negative pressure employed or due to the weight of the drainage fluid when filled. The container is preferably transparent and provided with graduation marks 27.

The seal 22 may be formed of any suitable material which will provide an adequate seal and which can be pierced by a spike. It may be a laminate including a plastic layer and a metal foil layer.

The cap 18 is preferably formed of an elastomeric material such as a natural or synthetic rubber or a plastic. Cap 18 includes a cylindrical portion 28 tightly fitting about neck 24 in a fluid tight sealing engagement. The container is shown having an annular flange 30 which enters a complementary annular groove on the interior of the cylindrical portion 28 of the cap to ensure a fixed relation between the cap 18 and container 16. The cylindrical portion 28 connects with a radially inwardly extending shoulder 32 which extends over the seal 22. Integrally connected with the shoulder 32 is an upper cylindrical portion 33 having an inner cylindrical passage or socket 34 which has internal radially inwardly extending seal rings 36 as will be further discussed hereafter. Cap 18 also includes an elastomeric stopper 38 integrally connected by a strap 40 and secured by a readily breakable connection 39, as shown in Figure 3. Stopper 38 is sized to enter and close the socket 34 after the bottle has been filled with drainage fluid. A flange 41, integral with the stopper, can be grasped by the fingers for breaking the connection 39 when it is desired to use the stopper. Cap 18 is also provided with an integral eye 42 for receiving a conventional adjustable connector strap 44 which may be used for connecting the bottle to a support, for example, to a bedrail or the like. The socket 34 of the cap is open and is in axially aligned relation with the seal 22 and the bore 26 of the neck 24.

The connecting strap 40 is long enough to permit the plug 38 to be inserted into the cap socket 34 so that the container, when filled, may be properly transported and discarded.

The connector assembly 20, as best seen in Figures 4 and 5, includes a generally inverted cup-shaped connector 50 having an upper generally flat wall 52 and a depending generally cylindrical sidewall or shield 54 integrally connected with the upper wall 52. Extending upwardly from the upper wall is a cylindrical fluid drainage inlet 56 having an inlet port or passage 58. Passage 58 connects with a bore or passage 60 of a depending hollow spike 62 integrally connected to the upper wall 52, the spike 62 extending from the centre of the inner side of the upper wall and generally concentrically with the shield or sidewall 54. The lower end of spike 60 is shown cut arcuately to form a pointed end 63 for piercing the bottle seal 22.

The connector 50 also includes an upwardly extending fluid pressure indicating port 64 having a passage 66 connected with the passage 60 of spike 62 by way of an opening 68 extending through the upper wall 52. A gas or air-pressure responsive, resiliently compressible bulb 69 has one end closed and the other end in sealing connection about the indicating port 64.

A flexible tube 70, which may be of a suitable plastic such as polyvinyl chloride, has its proximal end sealingly connected to the inlet port and its distal end sealingly connected (Figure 1) with the proximal end of drainage catheter 12. Tube 70 may be provided with any conventional or suitable tube clamp or clip, such as clip 72, that can be manually operated to selectively open and close tube 70. Clip 72 is shown in the open condition with the tube passing through an enlarged opening. When the clip is moved downwardly relative to the tube 70, the tube enters a reduced opening to clamp the lumen tube 70 closed.

Connector 50 may be formed or moulded of a suitable plastic which is essentially rigid, for example, it may be formed of an acrylic, relatively rigid polyvinyl chloride, or other plastic that provides sufficient rigidity. The side walls 54 may be slightly tapered radially outwardly in a downward direction to ensure that the sidewall or shield 54 does not effect an interference with the cylindrical portion 28 of the cap 18 during movement of the connector 50 onto the cap.

The container 16 is evacuated and sealed at a negative pressure suitable for the purpose. The cap 18 is applied to the bottle during manufacture and the assembly can be sold and stored for use as shown in Figure 2. The connector assembly 20 may be assembled by the manufacturer so that the connector 50, indicating bulb 69, and the inlet tube 70 are connected together and can be stored for use as a unitary assembly.

In use, when it is desired to connect an evacuated container 16 with a catheter, such as catheter 12, that has been introduced into a body wound, the distal end of tube 70 of the connector assembly 20 is connected to the proximal end of the catheter (Figure 1). Preferably, this connection is made with the tube clamp 72 moved to a tube pinching position to close the tube lumen. Then, the outer surface of the connector shield 54 may be grasped with one hand, and while securing the container 16 or holding it with the other hand, the spike 62 (Figure 4) of connector 50 is inserted into the upper open end of socket 34 of cap 18. During the insertion of spike 62, the shield 54 moves longitudinally over the sidewalls 28 of cap 18 and the spike pierces seal 22 and enters the bore 26 of neck 24 to effect fluid communication between the spike bore 60 and the interior of container 16. The piercing of seal 22 connects the pressure indicating passage 68, port 66 and indicator 69, as well as the passage 58 of the inlet port 56 to the interior of the bottle. When the tube clamp 72 is moved to open tube 70 (its position in Figure 1), the catheter 12 is connected in fluid communication with the interior of bottle 16 so that drainage fluid, such as gas and liquid, can flow into the tip of catheter 12, tube 70, spike passage 60 and into the interior of the container.

During insertion of spike 62 into the socket 34 of cap 18, the outer cylindrical surface of the spike is in sealing engagement with the socket 34 since the sealing rings 36 resiliently engage the surface of the spike prior to and after the spike has pierced the seal 22. With the construction shown, the elastomeric cap 18 tightly and sealingly fits about the neck 24 of the container and the spike 62 so that fluid can flow into the interior of the container only by way of the spike bore 60.

When it is desired to remove the container 16, such as when the container becomes filled with wound drainage fluid, the clamp 72 may be moved to clamp tube 70 closed and connector 50 may be pulled upwardly relative to container 16 to remove the spike 62 from cap 18 and completely disconnect the connector assembly 20. The stopper 38 of cap 18 may then be inserted into socket 34 of the cap to close the container. The container 16 may then be discarded. If drainage fluid is to be further collected, a new container 16 may be provided and the same connector assembly 20 connected to the fresh container without the necessity of disconnecting the tubing from the catheter. The new container may be connected in the same manner as the first bottle as described above. Thus, the same connector 50, for a given patient wound, can be connected and disconnected from any number of collection containers required.

Each time the connector 50 is connected to a container, the upper end of indicator bulb 69 becomes flattened due to the negative pressure within the container. That is, since the interior of the bulb 69 is connected in fluid communication with the interior of the container, the negative pressure will act on the interior walls of the bulb compressing the bulb or flattening it to thereby provide a visual indication that the interior of the container is at a negative pressure. Should the rubber bulb 69 fail to flatten after the connector has been inserted onto the container it will be immediately apparent that a partial vacuum or negative pressure does not exist so that another container must be used. Alternatively it may indicate that the spike has not been moved sufficiently to pierce the seal 22.

Because the spike shield 54 completely surrounds or covers the spike, the connector 50 can be handled without contaminating or touching the spike. Also, since all the drainage fluid flows through the bore 60 of the spike, the connector 50 can be readily removed from one bottle and connected with another bottle without drainage fluid contacting the person using the apparatus. Thus, the same connector assembly 20, for the same wound can be readily used with any number of collection bottles thereby providing an economical and efficient drainage device.

Furthermore, because the pressure indicating means 68 is disposed on the connector assembly 20 instead of on the collection bottle 16, the number of pressure indicating devices per patient requirement is substantially less since one connector assembly 20 may be used with a plurality of bottles.

As various changes could be made in the above construction without departing from the scope of the invention, it is intended that all matter contained in the above description and apparatus shown in the accompanying drawings shall be interpreted as illustrative and not in a limiting sense.

CLAIMS

1. Wound drainage apparatus comprising an evacuated collection container (16) having a pierceable seal (22) over an opening therein, a socket (34) defined about said seal, and a connector (20) including an inlet (56) for drainage fluid, a spike (62) for insertion into said socket (34) and adapted to pierce said seal (22) to connect the interior of the container with said inlet, and a shield (54) surrounding said spike (62).

2. Apparatus according to Claim 1, wherein said socket (34) includes sealing means (36) for engagement with said spike (62).

3. Apparatus according to Claim 1 or Claim 2, wherein said shield (54) encircles the spike (62) and has an axial length at least as great as that of the spike (62).

4. Apparatus according to Claim 3, wherein said shield (54) has an axial length greater than that of the spike (62).

5. Apparatus according to Claim 3 or Claim 4, wherein said shield (54) comprises a transverse wall having a sleeve depending therefrom which is adapted to surround said socket (34).

6. Apparatus according to Claim 5 wherein said connector includes an integral upstanding tubular projection defining said inlet (56) and an integral depending projection defining said spike (62), said inlet and spike being in fluid communication.

7. Apparatus according to any preceding claim wherein said spike (62) is tubular and pointed at the outer end thereof.

8. Apparatus according to any preceding claim wherein said connector is a single piece plastics moulding.

9. Apparatus according to any preceding claim wherein said container is a bottle having a neck (24), said seal (22) being over the opening thereof, a resilient sleeve (18) in elastic engagement on said neck upstanding therefrom to define said socket (34).

10. Apparatus according to Claim 9 wherein said sleeve (18) comprises a first cylindrical portion (28) to surround said neck (24), a second cylindrical portion (33) to define said socket (34) and an intermediate annular shoulder (32) connected at the outer edge thereof to said first portion (28) and at the inner edge thereof to said second portion (33).

11. Apparatus according to Claim 9 or 10, wherein a stopper (38) is integrally connected to the sleeve (18) for insertion into said socket (22).

12. Apparatus according to any preceding claim and further including pressure indicating means comprising a resilient bulb (69) in fluid communication with said inlet (56).

13. Apparatus according to any preceding claim wherein said seal (22) is a foil seal.

0162697

1/1

FIG.1

FIG.2

FIG.3

FIG.4

FIG.5